(19) Europäisches Patentamt
European Patent Office
Office européen des brevets

(11) Veröffentlichungsnummer: **0 149 237**
**A2**

(12) # EUROPÄISCHE PATENTANMELDUNG

(21) Anmeldenummer: **84116310.8**

(22) Anmeldetag: **27.12.84**

(51) Int. Cl.⁴: **C 07 D 241/06**
A 61 K 31/495

(30) Priorität: **12.01.84 DE 3400765**

(43) Veröffentlichungstag der Anmeldung:
**24.07.85 Patentblatt 85/30**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Wehinger, Egbert, Dr.**
**Gellertweg 33**
**D-5600 Wuppertal 1(DE)**

(72) Erfinder: **Kazda, Stanislav, Dr.**
**Gellertweg 18**
**D-5600 Wuppertal 1(DE)**

(54) **1.4-Dihydropyrazine, Verfahren zu ihrer Herstellung sowie ihre Verwendung in Arzneimitteln.**

(57) Die vorliegende Erfindung betrifft neue 1.4-Dihydropyrazine der allgemeinen Formel I

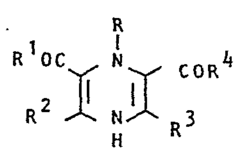

I

in welcher R, $R^1$, $R^2$, $R^3$ und $R^4$ die in der Beschreibung angegebene Bedeutung haben, mehrere Verfahren zu ihrer Herstellung sowie ihre Verwendung in Arzneimitteln, insbesondere in kreislaufbeeinflussenden Mitteln.

EP 0 149 237 A2

BAYER AKTIENGESELLSCHAFT 5090 Leverkusen, Bayerwerk

Konzernverwaltung RP
Patentabteilung

KS/m-c
Ib (Pha)

# 1.4-Dihydropyrazine, Verfahren zu ihrer Herstellung sowie ihr Verwendung in Arzneimitteln

Die vorliegende Erfindung betrifft neue 1.4-Dihydropyrazine, mehrere Verfahren zu ihrer Herstellung sowie ihre Verwendung in Arzneimitteln, insbesondere in kreislaufbeeinflussenden Mitteln.

Es ist bereits bekannt geworden, daß das 1.4-Dihydropyrazinsystem in einigen biochemisch wichtigen Molekülen wie z.B. den Flavocoenzymen oder bestimmten Luciferinen enthalten ist (vgl. C. Walsh, Acc.Chem.Res. 13, 148 (1980); T. Goto and Y. Kishi, Angew.Chem. 80, 417 (1968)).

Weiterhin ist schon bekannt, daß sich 1.4-Bis(trimethylsilyl)-1.4-dihydropyrazin durch reduktive Silylierung von Pyrazin mit Alkalimetallen und Halogensilanen darstellen läßt (R.A. Sulzbach und A.F.M. Iqbal, Angew. Chem. 83, 145 (1971)).

Die vorliegende Erfindung betrifft neue 1.4-Dihydropyrazine der allgemeinen Formel I,

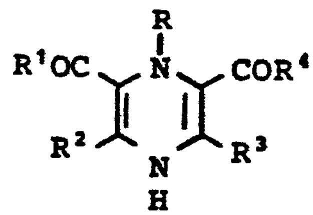

I

in welcher

R für carbocyclisches Aryl oder für Heterocyclen aus der Gruppe Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Benzthiadiazolyl, Chinolyl, Isochinolyl, Chinazolyl oder Chinoxalyl steht, wobei der Arylrest sowie die Heterocyclen gegebenenfalls 1 bis 3 gleiche oder verschiedene Substituenten aus der Gruppe Phenyl, Alkyl, Alkoxy, Alkylen, Dioxyalkylen, Halogen, Polyfluoralkyl, Polyfluoralkoxy, Alkylamino, Nitro, Cyano, Azido, Alkoxycarbonyl, Carbamoyl, Sulfamoyl oder $SO_m$-Alkyl ($m = 0$ bis 2) enthalten,

$R^1$ und $R^4$ gleich oder verschieden sind und

a) jeweils für eine Amino-, Mono- oder Dialkylaminogruppe stehen, wobei die Alkylgruppen gegebenenfalls durch einen Phenylrest substituiert sein können, oder

b) jeweils für einen gegebenenfalls durch Alkyl, Alkoxy oder Halogen substituierten Anilinorest stehen, oder

c) für die Reste $OR^5$ oder $OR^6$ stehen, wobei $R^5$ und $R^6$ für einen geradkettigen, verzweigten oder cyclischen, gesättigten oder ungesättigten Kohlenwasserstoffrest stehen, der gegebenenfalls durch ein Sauerstoff- oder Schwefelatom in der Kette unterbrochen ist, und/oder der gegebenenfalls substituiert ist durch Halogen, Cyano, Hydroxy, Nitro, Nitrooxy, Alkoxycarbonyl, Phenyl, Phenoxy, Phenylthio, Phenylsulfonyl, Pyridyl oder eine Aminogruppe, wobei diese Aminogruppe durch zwei gleiche oder verschiedene Substituenten aus der Gruppe Alkyl, Alkoxyalkyl, Aryl oder Aralkyl substituiert ist, oder wobei die Aminogruppe in der Weise substituiert ist, daß 2 Substituenten mit dem Stickstoffatom einen 5 bis 7 gliedrigen Ring bilden, der als weiteres Heteroatom Sauerstoff oder Schwefel oder eine N-Alkyl/Phenyl-Gruppierung enthalten kann,

und

$R^2$ und $R^3$ gleich oder verschieden sind und jeweils für Wasserstoff, einen geradkettigen oder verzweigten, gegebenenfalls substituierten Alkylrest, einen Arylrest oder einen Aralkylrest stehen.

Es wurde gefunden, daß man die erfindungsgemäßen 1.4-Dihydropyrazine der allgemeinen Formel I erhält, wenn man

A) 1. Hydrazone von α-Amino-ß-ketocarbonsäurederivaten der allgemeinen Formel II,

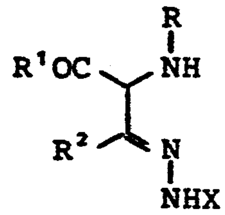

II

in welcher R, $R^1$ und $R^2$ die oben angegebene Bedeutung haben und X vorzugsweise für eine Carbamoyl- oder Alkoxycarbonylgruppe steht,

mit Azo-en-carbonylverbindungen der allgemeinen Formel III,

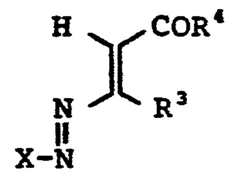

III

in welcher $R^3$ und $R^4$ die oben angegebene Bedeutung haben und X vorzugsweise für eine Carbamoyl- oder eine Alkoxycarbonylgruppe steht,

gegebenenfalls in Gegenwart inerter organischer Lösungsmittel umsetzt,

oder, nach dem gleichen Verfahren durch kreuzweisen Austausch der Substituenten $R^1$ und $R^4$ und der Substituenten $R^2$ und $R^3$, wenn man

2. Hydrazone von α-Amino-ß-ketocarbonsäurederivaten der allgemeinen Formel IV,

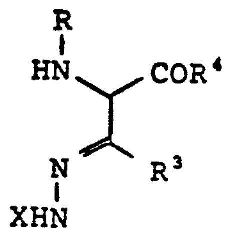

IV

in welcher R, $R^3$ und $R^4$ die oben angebene Bedeutung haben und X vorzugsweise für eine Carbamoyl- oder eine Alkoxycarbonylgruppe steht,

mit Azo-en-carbonylverbindungen der allgemeinen Formel V,

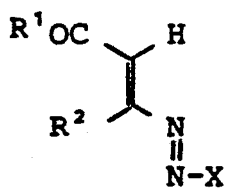

V

in welcher $R^1$ und $R^2$ die oben angegebene Bedeutung haben und X vorzugsweise für eine Carbamoyl- oder eine Alkoxycarbonylgruppe steht,

gegebenenfalls in Gegenwart inerter organischer Lösungsmittel umsetzt.

Weiterhin wurde gefunden, daß man für den Fall $R^1 = R^4$ und $R^2 = R^3$ in der allgemeinen Formel I die erfindungsgemäßen symmetrischen 1.4-Dihydropyrazine der allgemeinen Formel VI

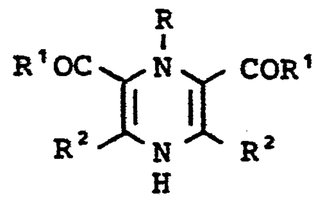

VI

erhält, wenn man

B) ein Mol der primären Amine der allgemeinen Formel VII,

$$R\text{-}NH_2 \qquad VII$$

in welcher R die oben angegebene Bedeutung hat,

mit zwei Mol der Azo-en-carbonylverbindung der allgemeinen Formel V,

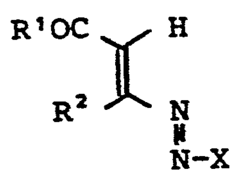

V

in welcher $R^1$, $R^2$ und X die oben angegebene Bedeutung haben,

gegebenenfalls in Gegenwart inerten organischer Lösungsmittel umsetzt.

Die erfindungsgemäßen 1.4-Dihydropyrazine sind neu und besitzen wertvolle pharmakologische Eigenschaften. Aufgrund ihrer kreislaufbeeinflussenden Wirkung können sie als antihypertensive Mittel, als Vasodilatatoren, als Cerebraltherapeutika sowie als Coronartherapeutika Verwendung finden und sind somit als Bereicherung der Pharmazie anzusehen.

Je nach Art der verwendeten Ausgangsstoffe kann die Synthese der erfindungsgemäßen Verbindungen durch folgende Formelschemata wiedergegeben werden, wobei die Herstellung von 1.4-Dihydro-2.6-dimethyl-4-(3-trifluormethylphenyl)-pyrazin-3.5-dicarbonsäure-isopropyl-methylester und 1.4-Dihydro-2.6-dimethyl-4-(3-nitrophenyl)-pyrazin-3.5-dicarbonsäure-isopropyl-methyl-ester sowie 1.4-Dihydro-2.6-dimethyl-4-(3-chlorphenyl)-pyrazin-3.5-dicarbonsäure-di(n)butylester als Beispiele gewählt seien:

A) 1.

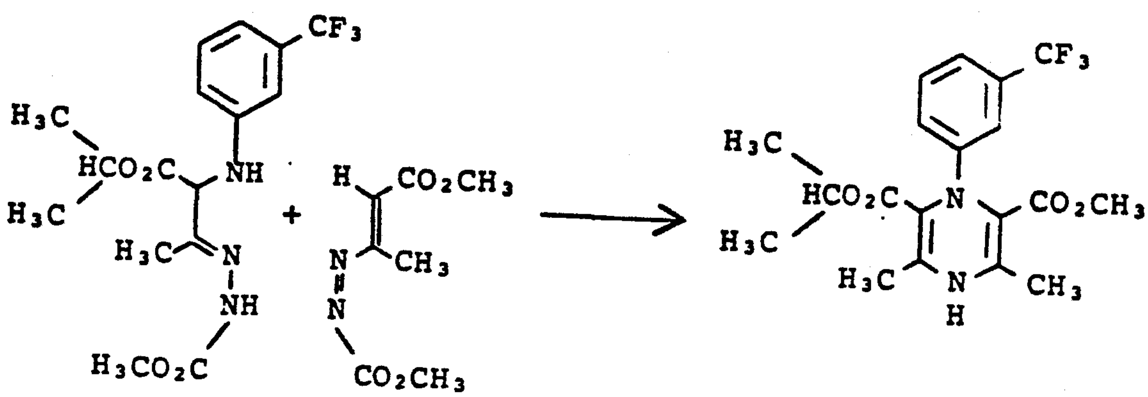

A) 2.

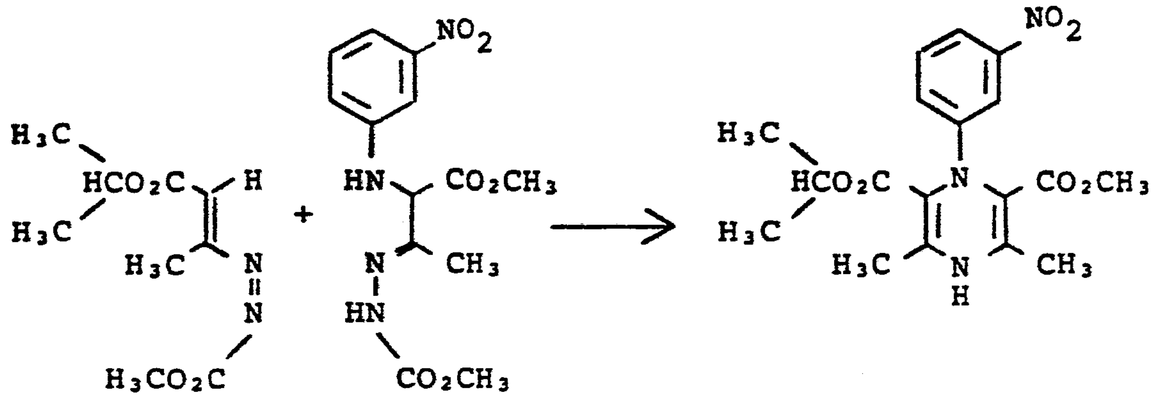

B)

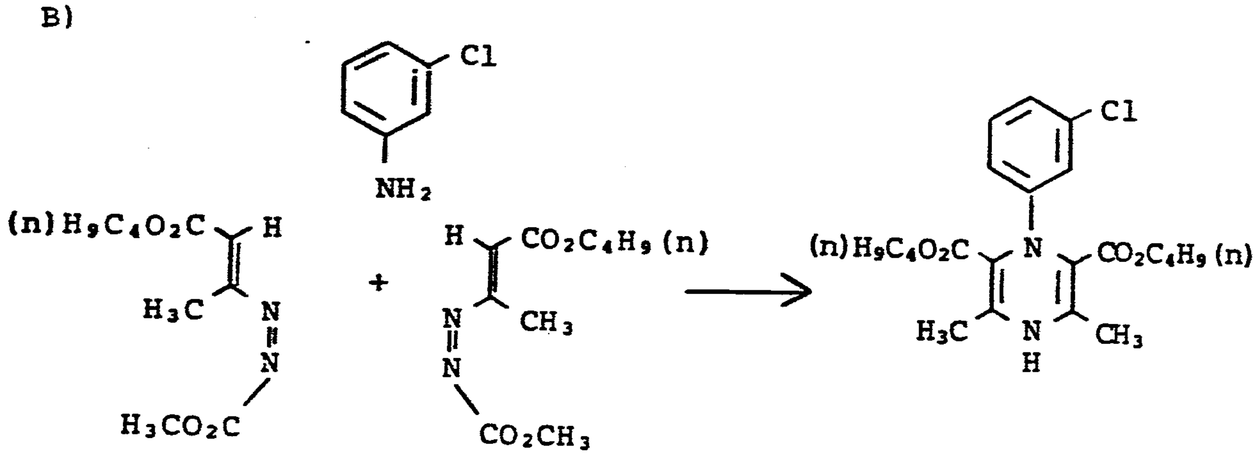

Verfahrensvariante A)1 und A)2

Gemäß Verfahren A)1 oder A)2 wird das Hydrazon eines α-Amino-ß-ketocarbonsäurederivates der allgemeinen Formel II oder der allgemeinen Formel IV

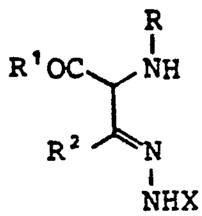

II

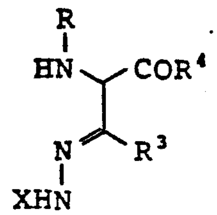

IV

mit einer Azo-en-carbonylverbindung der allgemeinen Formel III oder der allgemeinen Formel V

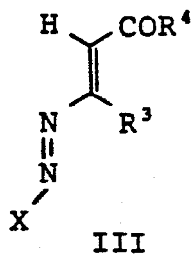

III

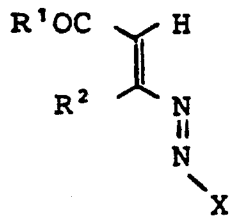

V

zur Reaktion gebracht.

In den Formeln II und IV und in der Formel I steht vorzugsweise

R für einen Phenyl- oder Naphthylrest oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxa-

0149237

zolyl, Benzisoxazolyl, Benzoxadiazolyl, Benzthiadiazolyl, Chinolyl, Isochinolyl, Chinazolyl oder Chinoxalyl, wobei die genannten Heterocyclen sowie der Phenylrest und der Naphthylrest 1 bis 3 gleiche oder verschiedene Substituenten tragen können, wobei als Substituenten Phenyl, geradkettiges oder verzweigtes Alkyl mit bis zu 8 Kohlenstoffatomen, Cycloalkyl mit 3 bis 7 Kohlenstoffatomen, Alkenyl oder Alkinyl mit 2 bis 6 Kohlenstoffatomen, Alkoxy mit 1 bis 4 Kohlenstoffatomen, Alkenoxy und Alkinoxy mit 2 bis 6 Kohlenstoffatomen, Tri-, Tetra- und Pentamethylen, Dioxymethylen, Dioxyethyliden, Halogen wie Fluor, Chlor, Brom oder Jod, Trifluormethyl, Trifluorethyl, Trifluormethoxy, Difluormethoxy, Tetrafluorethoxy, Dialkylamino mit 1 bis 4 C-Atomen, Nitro, Cyano, Azido, Alkoxycarbonyl mit 1 bis 4 C-Atomen im Alkoxyrest, Carbamoyl, N.N-Dimethylcarbamoyl, Sulfamoyl oder $SO_m$-Alkyl, worin m 0 oder 2 bedeutet und Alkyl 1 bis 4 Kohlenstoffatome enthält, aufgeführt seien.

Weiterhin stehen in den Formeln II und IV und in der Formel I vorzugsweise

$R^1$ und $R^4$, die gleich oder verschieden sind, für die Amino-, Mono- oder Dialkylaminogruppe mit bis zu 4 Kohlenstoffatomen je Alkylgruppe, die Benzylaminogruppe

oder

für den Anilinorest, der gegebenenfalls durch Fluor oder Chlor, oder durch Alkyl oder Alkoxy mit 1 bis 4 Kohlenstoffatomen substituiert ist,

für die Reste $OR^5$ oder $OR^6$, wobei $R^5$ und $R^6$ gleich oder verschieden sind und für einen geradkettigen, verzweigten oder cyclischen, gesättigten oder ungesättigten Kohlenwasserstoffrest mit bis zu 12 Kohlenstoffatomen stehen, der gegebenenfalls durch 1 Sauerstoff- oder Schwefelatom in der Kette unterbrochen ist und/oder der gegebenenfalls substituiert ist durch Halogen, Cyano, Hydroxy, Nitro, Nitrooxy, Alkoxycarbonyl mit bis zu 4 Kohlenstoffatomen in der Alkoxygruppe, Phenyl, Phenoxy, Phenylthio, Phenylsulfonyl, α-, ß-, γ-Pyridyl oder eine Aminogruppe, wobei diese Aminogruppe zwei gleiche oder verschiedene Substituenten aus der Gruppe Alkyl mit 1 bis 4 Kohlenstoffatomen, Alkoxyalkyl mit bis zu 6 Kohlenstoffatomen, Phenyl, Benzyl oder Phenethyl trägt oder wobei der Stickstoff dieser Aminogruppe mit den Substituenten einen 5- bis 7-gliedrigen Ring bildet, der als weiteres Heteroatom ein Sauerstoff- oder Schwefelatom oder eine N-Phenylgruppe oder eine N-Alkylgruppe mit 1 bis 4 Kohlenstoffatomen im Alkylrest enthalten kann,

$R^2$ und $R^3$, die gleich oder verschieden sind, für Wasserstoff, einen geradkettigen oder verzweigten Alkylrest mit bis zu 4 Kohlenstoffatomen, einen Phenylrest oder einen Benzylrest

und

X für die Gruppe $-CONH_2$ oder für die Gruppe $-COOR^7$, wobei $R^7$ einen geradkettigen oder verzweigten Alkylrest mit bis zu 5 Kohlenstoffatomen darstellt.

In den Formeln III und V haben die Reste $R^4$, $R^1$, $R^3$, $R^2$ und X die oben angegebene Bedeutung.

Die als Ausgangsstoffe verwendeten Hydrazone der α-Amino-ß-ketocarbonsäurederivate der allgemeinen Formel II oder IV sind literaturbekannt oder können nach literaturbekannten Methoden hergestellt werden (vgl. A.G. Schultz und W.K. Hagmann, J.Org.Chem. 43, 3391 (1978)).

Als Beispiele seien genannt:

2-Anilino-acetessigsäuremethylester-methoxycarbonylhydrazon, 2-(3-Methylanilino)-acetessigsäureethylester-methoxycarbonylhydrazon, 2-(2-Methylanilino)-acetessigsäurebutylester-methoxycarbonylhydrazon, 2-(2-Chloranilino)-acetessigsäureisopropylester-methoxycarbonylhydrazon, 2-(4-tert.Butylanilino)-acetessigsäure-tert.butylester-methoxycarbonylhydrazon, 2-(2-Methoxyanilino)-acetessigsäureisopropylester-methoxycarbonylhydrazon, 2-(Tetramethylenanilino)-acetessigsäuremethylester-methoxycarbonylhydrazon, 2-(3.4-Dioxymethylenanilino)-acetessigsäuredecylestermethoxycarbonylhydrazon, 2-(2.3-Dichloranilino)-acetessigsäuremethylester-methoxycarbonylhydrazon, 2-(2.4-Dichloranilino)-acetessigsäureethylester-methoxycarbonylhydrazon, 2-(2-Chloranilino)-acetessigsäure-(2.2.2-trifluorethyl)-ester-methoxycarbonylhydrazon, 2-(2-Trifluormethylanilino)-acetessigsäure-(2-methoxyethyl)-ester-methoxycarbonylhydrazon, 2-(2-Trifluormethoxyanilino)-acetessigsäureethylester-methoxycarbonylhydrazon, 2-(4-Dimethylaminoanilino)-

acetessigsäure-(2-methylthioethyl)-ester-methoxycarbonylhydrazon, 2-(3-Nitroanilino)-acetessigsäureisopropylester-methoxycarbonylhydrazon, 2-(2-Nitroanilino)-acetessigsäureisobutylester-methoxycarbonylhydrazon, 2-(2-Nitroanilino)-acetessigsäuremethylester-methoxycarbonylhydrazon, 2-(3-Nitroanilino)-acetessigsäure-(2-methoxyethyl)-ester-methoxycarbonylhydrazon, 2-(3-Nitroanilino)-acetessigsäurebenzylester-methoxycarbonylhydrazon, 2-(3-Nitroanilino)-acetessigsäure-(2-phenoxyethyl)-ester-methoxycarbonylhydrazon, 2-(3-Nitroanilino)-acetessigsäure(2-(N-Benzyl-N-methylamino)-ethyl)-ester-methoxycarbonylhydrazon, 2-(3-Cyanoanilino)-acetessigsäure-(2-cyanoethyl)-ester-methoxycarbonylhydrazon, 2-(3-Nitroanilino)-acetessigsäure-(2-nitroethyl)-ester-methoxycarbonylhydrazon, 2-(3-Nitroanilino)-acetessigsäure-(2-nitrooxyethyl)-ester-methoxycarbonylhydrazon, 2-(3-Nitroanilino)-acetessigsäure-(α-methoxycarbonyl-benzyl)-ester-methoxycarbonylhydrazon, 2-(3-Methylsulfonylanilino)-acetessigsäure-(α-pyridylmethyl)-ester-methoxycarbonylhydrazon, 2-(3-Chloranilino)-acetessigsäuredimethylamid-methoxycarbonylhydrazon, 2-(3-Nitroanilino)-acetessigsäure-(4-chloranilid)-methoxycarbonylhydrazon, 2-(3-Chloranilino)-acetessigsäureisopropylester-ethoxycarbonylhydrazon, 2-(2-Chloranilino)-acetessigsäureethylester-butoxycarbonylhydrazon, 2-(3-Trifluormethylanilino)-acetessigsäureethylester-tert.butoxycarbonylhydrazon, 2-(3-Nitroanilino)-acetessigsäuremethylester-carbamoylhydrazon, 2-(2-Nitroanilino)-acetessigsäureisobutylester-carbamoylhydrazon.

Die als Ausgangsstoffe verwendeten Azo-en-carbonylverbindungen der allgemeinen Formel III oder V sind literaturbekannt oder können nach literaturbekannten Methoden hergestellt werden (vgl. A.G. Schultz und W.K. Hagmann, J.Org.Chem. 43, 3391 (1978)).

Als Beispiele seien genannt:

3-(Methoxycarbonylazo)-crotonsäuremethylester, 3-(Methoxycarbonylazo)-crotonsäurebutylester, 3-(Methoxycarbonylazo)-crotonsäuredecylester, 3-(Methoxycarbonylazo)-crotonsäureisopropylester, 3-(Methoxycarbonylazo)-crotonsäure-tert.butylester, 3-(Methoxycarbonylazo)-crotonsäurecyclopentylester, 3-(Methoxycarbonylazo)-crotonsäurebenzylester, 3-(Methoxycarbonylazo)-crotonsäure-(2-methoxyethyl)-ester, 3-(Methoxycarbonylazo)-crotonsäure-(2-phenoxyethyl)-ester, 3-(Methoxycarbonylazo)-crotonsäure-(2-cyanoethyl)-ester, 3-(Methoxycarbonylazo)-crotonsäure-(2-chlorethyl)-ester, 3-(Methoxycarbonylazo)-crotonsäure-(2-cyanoethyl)-ester, 3-(Methoxycarbonylazo)-crotonsäure-(2-nitrooxyethyl)-ester, 3-(Methoxycarbonylazo)-crotonsäure-(2-methoxycarbonylethyl)-ester, 3-(Methoxycarbonylazo)-crotonsäure-(4-pyridylmethyl)-ester, 3-(Methoxycarbonylazo)-crotonsäure-(2-dimethylaminoethyl)-ester, 3-(Methoxycarbonylazo)-crotonsäure-(2-(N-benzyl-N-methylamino)-ethyl)-ester, 3-(tert.Butoxycarbonylazo)-crotonsäuremethylester, 3-(tert.Butoxycarbonylazo)-crotonsäureisopropylester, 3-(tert.Butoxycarbonylazo)-crotonsäure-(2-methoxyethyl)-ester, 3-(Carbamoylazo)-crotonsäureethylester, 3-(Carbamoylazo)-crotonsäure-(2-methoxyethyl)-ester.

Als Verdünnungsmittel kommen alle inerten Lösungsmittel in Frage. Hierzu gehören vorzugsweise Alkohole wie Methanol, Ethanol oder Isopropanol, Ether wie Dioxan, Tetrahydrofuran oder Glykolmonomethylether, Dimethylformamid, Dimethylsulfoxid, Acetonitril, Pyridin oder Hexamethylphosphorsäuretriamid.

Die Reaktionstemperaturen können in einem größeren Bereich variiert werden. Im allgemeinen arbeitet man zwischen 20° und 150°C, vorzugsweise bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens wird ein Mol des Hydrazons des α-Amino-ß-ketocarbonsäurederivates der allgemeinen Formel II oder IV mit etwa einem Mol Azo-en-carbonylverbindung der allgemeinen Formel III oder V in einem geeigneten Lösungsmittel zur Reaktion gebracht. Die Isolierung und Reinigung der erfindungsgemäßen Substanzen erfolgt vorzugsweise derart, daß man das Lösungsmittel im Vakuum abdestilliert und den Rückstand gegebenenfalls den aus dem Stand der Technik her bekannten Reinigungsoperationen wie der Umkristallisation oder der chromatographischen Trennung unterwirft.

Verfahrensvariante B

Gemäß Verfahren B wird ein primäres Amin der allgemeinen Formel VII

$$R\text{-}NH_2 \qquad VII$$

mit einer Azo-en-carbonylverbindung der allgemeinen Formel V

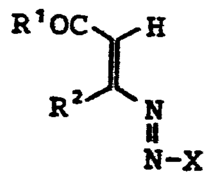

V

zur Reaktion gebracht.

In den Formeln VII und V haben die Reste R, $R^1$, $R^2$ und X die oben angegebene Bedeutung.

Die als Ausgangssubstanzen verwendeten Amine der allgemeinen Formel VII sind literaturbekannt oder können nach literaturbekannten Methoden hergestellt werden (vgl. Houben-Weyl, Methoden der organischen Chemie, Bd. XI, 4. Auflage 1957).

Als Beispiele seien genannt:

Anilin, α- oder β-Naphthylamin, 2-Chloranilin, 2.3-Dichloranilin, 2.6-Dichloranilin, o-Toluidin, 2-Methoxyanilin, 3.4-Dioxymethylenanilin, 4-tert.Butylanilin, 2-Cyclopropylanilin, 2.3-Tetramethylenanilin, 2-Fluoranilin, 3-Chlor-2-fluoranilin, 3-Jodanilin, 2-Trifluormethylanilin, 3-Trifluormethylanilin, 2-Trifluormethoxyanilin, 2-Difluormethoxyanilin, 4-Dimethylaminoanilin, 2-Nitroanilin, 3-Nitroanilin, 3-Cyanoanilin, 2-Methylthioanilin, 3-Methylsulfonylanilin, 2-Aminopyridin, 3-Aminopyridin, 2-Aminochinolin, Aminobenzoxadiazol, Aminobenzthiadiazol.

Die als Ausgangsstoffe verwendeten Azo-en-carbonylverbindungen der allgemeinen Formel V wurden bereits unter der Verfahrensvariante A und B angegeben.

Als Verdünnungsmittel kommen alle inerten Lösungsmittel in Frage. Hierzu gehören vorzugsweise Alkohole wie Methanol, Ethanol oder Isopropanol, Ether wie Dioxan, Tetrahydrofuran oder Glykolmonomethylether, Dimethylformamid, Dimethylsulfoxid, Acetonitril, Pyridin oder Hexamethylphosphorsäuretriamid.

Die Reaktionstemperaturen können in einem größeren Bereich variiert werden. Im allgemeinen arbeitet man zwischen 20° und 150°C, vorzugsweise bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens wird ein Mol des primären Amins der allgemeinen Formel VII mit etwa zwei Mol der Azo-en-carbonylverbindung der allgemeinen Formel V in einem geeigneten Lösungsmittel zur Reaktion gebracht. Die Isolierung und Reinigung der erfindungsgemäßen Substanzen erfolgt vorzugsweise derart, daß man das Lösungsmittel im Vakuum abdestilliert und den Rückstand gegebenenfalls den aus dem Stand der Technik her bekannten Reinigungsoperationen wie der Umkristallisation aus einem geeigneten Lösungsmittel oder der chromatographischen Trennung unterwirft.

Die vorstehenden Herstellungsverfahren sind lediglich zur Verdeutlichung angegeben, und die Herstellung der Verbindungen der allgemeinen Formel I ist nicht auf diese Verfahren beschränkt, sondern jede Modifikation dieser Verfahren ist in gleicher Weise für die Herstellung der erfindungsgemäßen Verbindungen anwendbar.

Von besonderem Interesse sind Verbindungen der allgemeinen Formel I, in welcher

R für Phenyl, Naphthyl, Pyridyl, Benzoxadiazolyl oder Benzthiadiazolyl steht, wobei der Phenylrest gegebenenfalls durch 1, 2 oder 3 gleiche oder verschiedene Substituenten aus der Gruppe Alkyl mit 1 bis 4 C-Atomen, Alkoxy mit 1 bis 4 C-Atomen, Dioxymethylen, Halogen, Trifluormethyl, Difluormethoxy, Dialkylamino mit 1 bis 4 C-Atomen, Nitro, Cyano, Azido und Alkoxycarbonyl mit 1 bis 4 C-Atomen im Alkoxyrest substituiert ist,

$R^1$ und $R^4$ gleich oder verschieden sind und jeweils für $OR^5$ oder $OR^6$ stehen, wobei $R^5$ und $R^6$ gleich oder verschieden sind und für einen geradkettigen, verzweigten oder cyclischen, gesättigten oder ungesättigten Kohlenwasserstoffrest mit bis zu 12 Kohlenstoffatomen stehen, der gegebenenfalls durch ein Sauerstoff der Kette unterbrochen ist und/oder der gegebenenfalls substituiert ist durch Halogen, Cyano, Hydroxy, Nitro, Nitrooxy, Alkoxycarbonyl mit bis zu 4 C-Atomen in der Alkoxygruppe, Phenyl oder eine Aminogruppe, die zwei gleiche oder verschiedene Substituenten aus der Gruppe Alkyl mit 1 bis 4 C-Atomen oder Benzyl trägt oder wobei der Stickstoff dieser Aminogruppe mit seinen Substituenten einen 5 bis 7 gliedrigen Ring bilden, der als weiteres Heteroatom ein Sauerstoff- oder eine N-Alkylgruppe mit 1 bis 4 C-Atomen im Alkylrest enthalten kann, und

$R^2$ und $R^3$ gleich oder verschieden sind und für Wasserstoff, Alkyl mit 1 bis 4 Kohlenstoffatomen, Phenyl oder Benzyl stehen.

Besonders hervorgehoben seien Verbindungen der allgemeinen Formel I, in welcher

R für Phenyl, Pyridyl, Benzoxadiazolyl oder Benzthiadiazolyl steht, wobei der Phenylrest gegebenenfalls 1, 2 oder 3 gleiche oder verschiedene Substituenten aus der Gruppe Alkyl mit 1 bis 4 C-Atomen, Alkoxy mit 1 bis 4 C-Atomen, Fluor, Chlor, Trifluormethyl,

Trifluormethoxy oder Carbalkoxy mit 1 bis 4 C-Atomen im Alkoxyrest, trägt,

$R^1$ und $R^4$ gleich oder verschieden sind und für die Reste $OR^5$ oder $OR^6$ stehen, wobei $R^5$ und $R^6$ gleich oder verschieden sind und für einen geradkettigen, verzweigten oder cyclischen, gesättigten Kohlenwasserstoffrest mit bis zu 12 Kohlenstoffatomen stehen, der gegebenenfalls durch ein Sauerstoff in der Kette unterbrochen ist und/oder der gegebenenfalls substituiert ist durch Fluor, Chlor, Cyano, Hydroxy oder Nitro und

$R^2$ und $R^3$ gleich oder verschieden sind und für Wasserstoff, Alkyl mit 1 bis 4 Kohlenstoffatomen oder Benzyl stehen.

Außer den unten aufgeführten Herstellungsbeispielen seien folgende erfindungsgemäßen Wirkstoffe genannt:

1.4-Dihydro-2.6-dimethyl-4-(2-methylphenyl)-pyrazin-3.5-dicarbonsäure-dipropylester

1.4-Dihydro-2.6-dimethyl-4-(2-chlorphenyl)-pyrazin-3.5-dicarbonsäure-ethyl-methyl-ester

1.4-Dihydro-2.6-dimethyl-4-(2-chlorphenyl)-pyrazin-3.5-dicarbonsäure-methyl-(2.2.2-trifluorethyl)-ester

1.4-Dihydro-2.6-dimethyl-4-(2-trifluormethylphenyl)-pyrazin-3.5-dicarbonsäurediethylester

0149237

1.4-Dihydro-2.6-dimethyl-4-(2-nitrophenyl)-pyrazin-3.5-dicarbonsäuredimethylester

1.4-Dihydro-2.6-dimethyl-4-(2-nitrophenyl)-pyrazin-3.5-dicarbonsäure-isobutyl-methyl-ester

1.4-Dihydro-2.6-dimethyl-4-(3-nitrophenyl)-pyrazin-3.5-dicarbonsäure-ethyl-methyl-ester

1.4-Dihydro-2.6-dimethyl-4-(3-nitrophenyl)-pyrazin-3.5-dicarbonsäure-isopropyl-(2-methoxyethyl)-ester

1.4-Dihydro-2.6-dimethyl-4-(3-nitrophenyl)-pyrazin-3.5-dicarbonsäure-decyl-ethyl-ester

1.4-Dihydro-2.6-dimethyl-4-(3-nitrophenyl)-pyrazin-3.5-dicarbonsäure-isopropyl-(2-propoxyethyl)-ester

1.4-Dihydro-2.6-dimethyl-4-(3-nitrophenyl)-pyrazin-3.5-dicarbonsäure-(2-(N-benzyl-N-methylamino)-ethyl)-methyl-ester

1.4-Dihydro-2.6-dimethyl-4-(2.1.3-benzoxadiazolyl-4)-pyrazin-3.5-dicarbonsäurediethylester

1.4-Dihydro-2.6-dimethyl-4-(2.1.3-benzoxadiazolyl-4)-pyrazin-3.5-dicarbonsäure-isopropyl-methyl-ester

1.4-Dihydro-2.6-dimethyl-4-(2.3-dichlorphenyl)-pyrazin-3.5-dicarbonsäure-ethyl-methyl-ester

1.4-Dihydro-2.6-dimethyl-4-(3-chlorphenyl)-3-carbamoyl-5-pyrazincarbonsäuremethylester

1.4-Dihydro-2.6-dimethyl-4-(3-nitrophenyl)-pyrazin-3.5-dicarbonsäure-isopropyl-(2-methoxy-2-phenylethyl)-ester

1.4-Dihydro-2-ethyl-6-methyl-4-(2-cyanophenyl)-pyrazin-3.5-dicarbonsäurediethylester

1.4-Dihydro-2.6-diethyl-4-(3.4.5-trichlorphenyl)-pyrazin-3.5-dicarbonsäure-isopropyl-methyl-ester

1.4-Dihydro-2-isopropyl-6-methyl-4-(3-nitrophenyl)-pyrazin-3.5-dicarbonsäuredimethylester

1.4-Dihydro-2-benzyl-6-methyl-4-(3-nitrophenyl)-pyrazin-3.5-dicarbonsäurediethylester

1.4-Dihydro-2.6-dimethyl-4-(pyridyl-2)-pyrazin-3.5-dicarbonsäuredimethylester

Die neuen Verbindungen haben ein breites und vielseitiges pharmakologisches Wirkungsspektrum.

Im einzelnen konnten im Tierexperiment folgende Hauptwirkungen nachgewiesen werden:

1. Die Verbindungen bewirken bei parenteraler, oraler und perlingualer Zugabe eine deutliche und langanhaltende Erweiterung der Coronargefäße. Diese Wirkung auf die Coronargefäße wird durch einen gleichzeitigen nitritähnlichen herzentlastenden Effekt verstärkt. Sie beeinflussen bzw. verändern den Herzstoffwechsel im Sinne einer Energieersparnis.

2. Die Erregbarkeit des Reizbildungs- und Erregungsleitungssystems innerhalb des Herzens wird herabgesetzt, so daß eine in therapeutischen Dosen nachweisbare Antiflimmerwirkung resultiert.

3. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z.B. dem Zentralnervensystem) manifestieren. Die Verbindungen eignen sich daher besonders als Cerebraltherapeutika.

4. Die Verbindungen senken den Blutdruck von normotonen und hypertonen Tieren und können somit als antihypertensive Mittel verwendet werden.

5. Verbindungen haben stark muskulär-spasmolytische Wirkungen, die an der glatten Muskulatur des Magens, Darmtrakts, des Urogenitaltraktes und des Respirationssystems deutlich werden.

Die erfindungsgemäßen Verbindungen eignen sich aufgrund dieser Eigenschaften besonders zur Prophylaxe und Therapie der akuten und chronischen ischämischen Herzkrankheit im weitesten Sinne, zur Therapie des Hochdrucks sowie zur Behandlung von cerebralen und peripheren Durchblutungsstörungen.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B.

Erdnuß-/Sesam-Öl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,05 bis 10 mg/kg, vorzugsweise etwa 0,1 bis 5 mg/kg Körpergewicht pro Tag zur Erzielung wirksamer Ergebnisse zu verabreichen, und bei oraler Applikation beträgt die Dosierung etwa 0,1 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

## Herstellungsbeispiele

Beispiel 1 (Verfahrensvariante A)

1.4-Dihydro-2.6-dimethyl-4-phenyl-pyrazin-3.5-dicarbonsäure-dimethylester

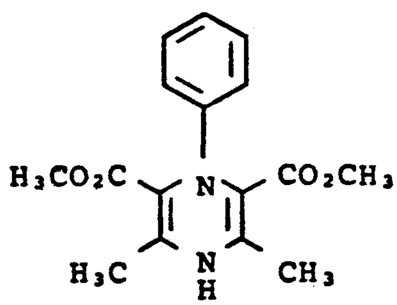

10.7 g (38.3 mMol) 2-Anilino-acetessigsäuremethylester-methoxycarbonylhydrazon wurden zusammen mit 7.13 g (38.3 mMol) 3-(Methoxycarbonylazo)-crotonsäuremethylester in 80 ml Ethanol 24 Stunden unter Rückfluß erhitzt. Anschließend wurde das Lösungsmittel im Vakuum abdestilliert und der Rückstand chromatographisch über Kieselgel mit einem Gemisch von Chloroform:Essigsäureethylester = 2:1 als Eluierungsmittel gereinigt.

Schmelzpunkt: 258°C
Ausbeute: 2.5 g (22 %).

Beispiel 2 (Verfahrensvariante B)

1.4-Dihydro-2.6-dimethyl-4-phenyl-pyrazin-3.5-dicarbon-säure-diethylester

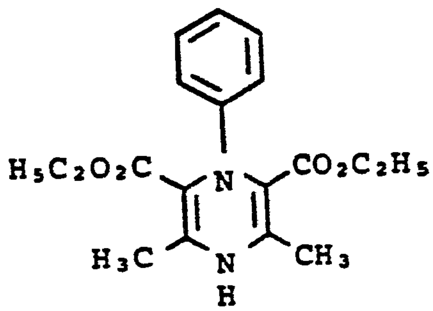

12.1 g (130 mMol) Anilin wurden zusammen mit 52 g (260 mMol) 3-(Methoxycarbonylazo)-crotonsäureethylester in 500 ml Ethanol aufgenommen und nach Beendigung der ersten exothermen Reaktion 24 Stunden unter Rückfluß erhitzt. Anschließend wurde das Lösungsmittel im Vakuum bis auf ca. 150 ml eingeengt, das ausgefallene Produkt abgesaugt und über Kieselgel/Chloroform:Essigester = 2:1 gereinigt. Umkristallisation aus Ethanol lieferte 10 g (23 %) analysenreines Produkt vom Schmelzpunkt Fp = 228°C.

Beispiel 3

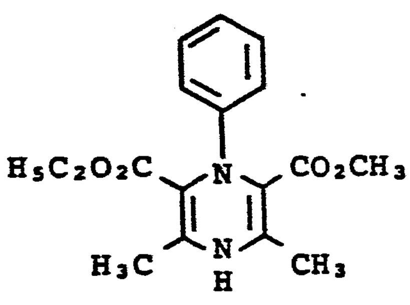

Analog Beispiel 1 wurde durch Umsetzung von 2-Anilinoacetessigsäureethylester-methoxycarbonylhydrazon mit 3-(Methoxycarbonylazo)-crotonsäuremethylester in Ethanol der 1.4-Dihydro-2.6-dimethyl-4-phenyl-pyrazin-3.5-dicarbonsäure-ethyl-methyl-ester vom Fp.: 235°C (Ethanol) erhalten.
Ausbeute: 25 %.

Beispiel 4

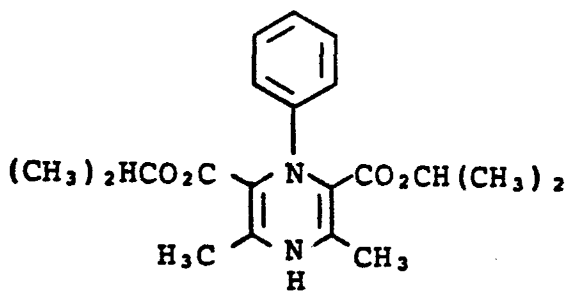

Analog Beispiel 1 wurde durch Umsetzung von 2-Anilinoacetessigsäureisopropylester-methoxycarbonylhydrazon mit 3-(Methoxycarbonylazo)-crotonsäureisopropylester in Ethanol der 1.4-Dihydro-2.6-dimethyl-4-phenyl-pyrazin-3.5-dicarbonsäurediisopropylester vom Fp: 198°C (Ethanol) erhalten.
Ausbeute: 19 %.

Beispiel 5

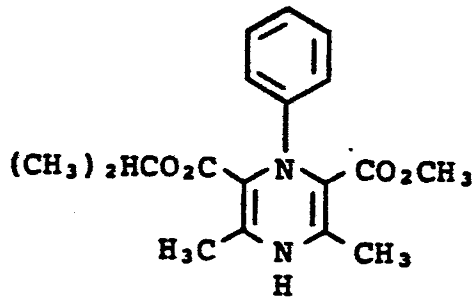

Analog Beispiel 1 wurde durch Umsetzung von 2-Anilino-acetessigsäuremethylester-methoxycarbonylhydrazon mit 3-(Methoxycarbonylazo)-crotonsäureisopropylester in Ethanol der 1.4-Dihydro-2.6-dimethyl-4-phenyl-pyrazin-3.5-dicarbonsäure-isopropyl-methylester vom Fp: 204°C (Ethanol) erhalten.
Ausbeute: 30 %

Beispiel 6

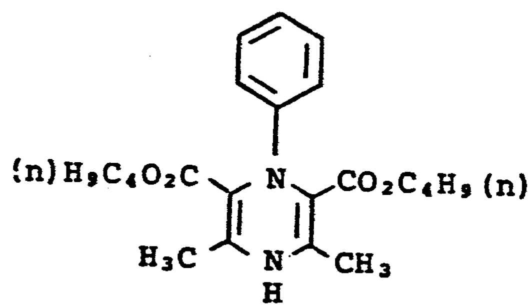

Analog Beispiel 1 wurde durch Umsetzung von 2-Anilinoacetessigsäurebutylester-methoxycarbonylhydrazon mit 3-(Methoxycarbonylazo)-crotonsäurebutylester in Ethanol der 1.4-Dihydro-2.6-dimethyl-4-phenyl-pyrazin-3.5-dicarbonsäuredibutylester vom Fp: 152°C (Ethanol) erhalten.
Ausbeute: 26 %

Beispiel 7

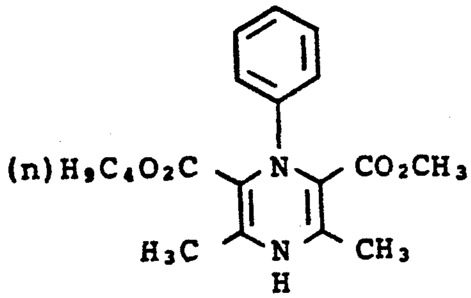

Analog Beispiel 1 wurde durch Umsetzung von 2-Anilino-acetessigsäuremethylester-methoxycarbonylhydrazon mit 3-(Methoxycarbonylazo)-crotonsäurebutylester in Ethanol der 1.4-Dihydro-2.6-dimethyl-4-phenyl-pyrazin-3.5-dicarbonsäure-butyl-methyl-ester vom Fp: 178°C (Ethanol) erhalten.
Ausbeute: 29 %

Beispiel 8

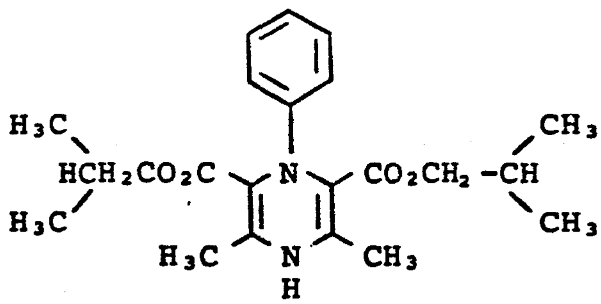

Analog Beispiel 1 wurde durch Umsetzung von 2-Anilinoacetessigsäureisobutylester-methoxycarbonylhydrazon mit 3-(Methoxycarbonylazo)-crotonsäureisobutylester in Ethanol der 1.4-Dihydro-2.6-dimethyl-4-phenyl-pyrazin-3.5-dicarbonsäurediisobutylester vom Fp: 181°C (Ethanol) erhalten.
Ausbeute: 19 %

Beispiel 9

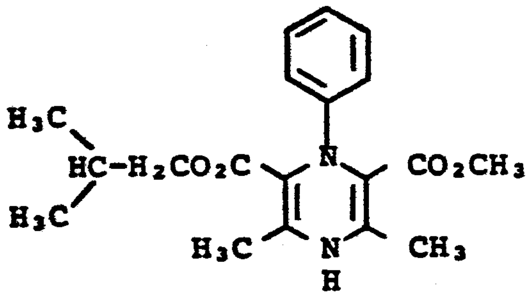

Analog Beispiel 1 wurde durch Umsetzung von 2-Anilino-acetessigsäuremethylester-methoxycarbonylhydrazon mit 3-(Methoxycarbonylazo)-crotonsäureisobutylester in Ethanol der 1.4-Dihydro-2.6-dimethyl-4-phenyl-pyrazin-3.5-dicarbonsäure-isobutyl-methylester vom Fp: 183°C (Ethanol) erhalten.
Ausbeute: 29 %.

Beispiel 10

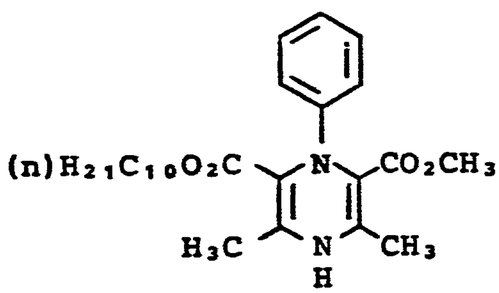

Analog Beispiel 1 wurde durch Umsetzung von 2-Anilino-acetessigsäuremethylester-methoxycarbonylhydrazon mit 3-(Methoxycarbonylazo)-crotonsäuredecylester in Ethanol der 1.4-Dihydro-2.6-dimethyl-4-phenyl-pyrazin-3.5-dicarbonsäure-decyl-methyl-ester vom Fp: 154°C (Ethanol) erhalten.
Ausbeute: 22 %.

Beispiel 11

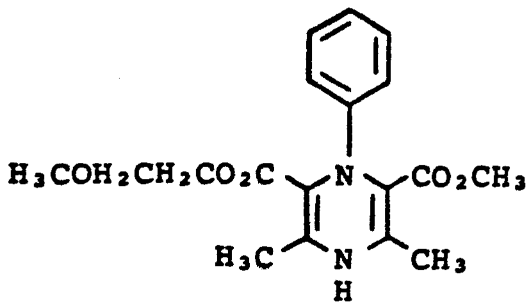

Analog Beispiel 1 wurde durch Umsetzung von 2-Anilino-acetessigsäuremethylester-methoxycarbonylhydrazon mit 3-(Methoxycarbonylazo)-crotonsäure-(2-methoxyethyl)-ester in Ethanol der 1.4-Dihydro-2.6-dimethyl-4-phenyl-pyrazin-3.5-dicarbonsäure-(2-methoxyethyl)-methyl-ester vom Fp: 157°C (Ethanol) erhalten.
Ausbeute: 15 %.

Beispiel 12

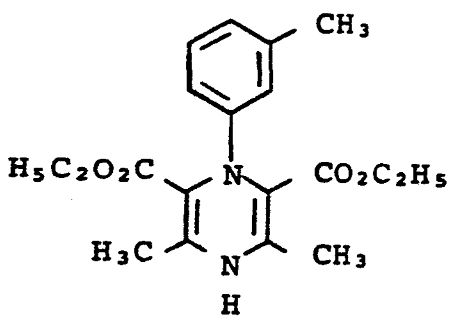

Analog Beispiel 2 wurde durch Umsetzung von 3-Methylanilin mit 3-(Methoxycarbonylazo)-crotonsäureethylester in Ethanol der 1.4-Dihydro-2.6-dimethyl-4-(3-methylphenyl)-pyrazin-3.5-dicarbonsäurediethylester vom Fp: 176°C erhalten.
Ausbeute: 16 %.

Beispiel 13

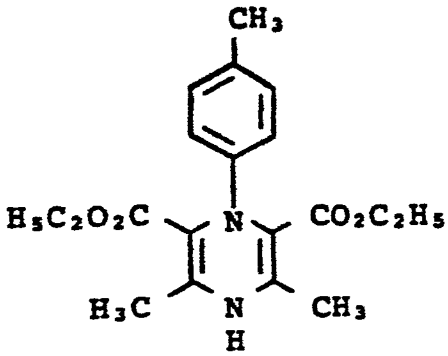

Analog Beispiel 1 wurde durch Umsetzung von 2-(4-Methyl-anilino)-acetessigsäureethylester-methoxycarbonylhydrazon mit 3-(Methoxycarbonylazo)-crotonsäureethylester in Ethanol der 1.4-Dihydro-2.6-dimethyl-4-(4-methylphenyl)-pyrazin-3.5-dicarbonsäurediethylester vom Fp: 211°C (Ethanol) erhalten.
Ausbeute: 32 %.

Beispiel 14

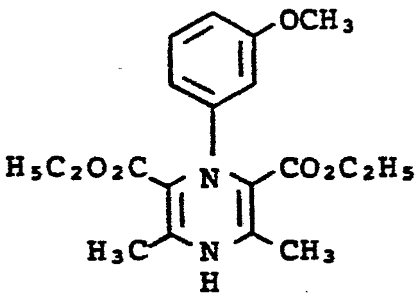

Analog Beispiel 1 wurde durch Umsetzung von 2-(3-Methoxy-anilino)-acetessigsäureethylester-methoxycarbonylhydrazon mit 3-(Methoxycarbonylazo)-crotonsäureethylester in Ethanol der 1.4-Dihydro-2.6-dimethyl-4-(3-methoxyphenyl)-pyrazin-3.5-dicarbonsäurediethylester vom Fp: 195°C erhalten.
Ausbeute: 21 %.

Beispiel 15

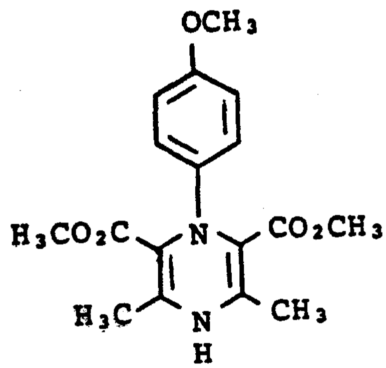

Analog Beispiel 1 wurde durch Umsetzung von 2-(4-Methoxy-anilino)-acetessigsäuremethylester-methoxycarbonylhydrazon mit 3-(Methoxycarbonylazo)-crotonsäuremethylester in Ethanol der 1.4-Dihydro-2.6-dimethyl-4-(4-methoxyphenyl)-pyrazin-3.5-dicarbonsäuredimethylester vom Fp: 200°C (Ethanol) erhalten.

Ausbeute: 26 %.

Beispiel 16

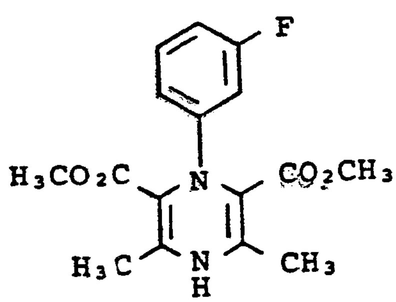

Analog Beispiel 1 wurde durch Umsetzung von 2-(3-Fluor-anilino)-acetessigsäuremethylester-methoxycarbonylhydrazon mit 3-(Methoxycarbonylazo)-crotonsäuremethylester in Ethanol der 1.4-Dihydro-2.6-dimethyl-4-(3-fluorphenyl)-pyrazin-3.5-dicarbonsäuredimethylester vom Fp: 229°C erhalten.

Ausbeute: 18 %.

Beispiel 17

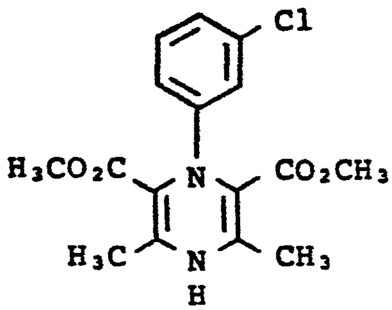

Analog Beispiel 2 wurde durch Umsetzung von 3-Chloranilin mit 3-(Methoxycarbonylazo)-crotonsäuremethylester in Ethanol der 1.4-Dihydro-2.6-dimethyl-4-(3-chlorphenyl)-pyrazin-3.5-dicarbonsäuredimethylester vom Fp: 200°C erhalten.
Ausbeute: 15 %.

Beispiel 18

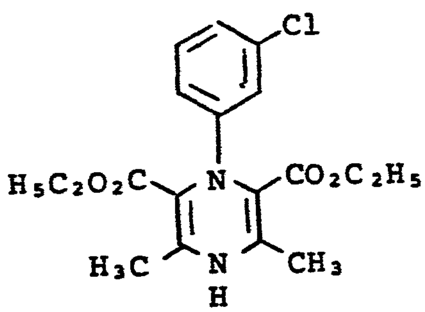

Analog Beispiel 1 wurde durch Umsetzung von 2-(3-Chloranilino)-acetessigsäureethylester-methoxycarbonylhydrazon mit 3-(Methoxycarbonylazo)-crotonsäureethylester in Ethanol der 1.4-Dihydro-2.6-dimethyl-4-(3-chlorphenyl)-pyrazin-3.5-dicarbonsäurediethylester vom Fp: 214°C (Ethanol) erhalten.
Ausbeute: 25 %.

Beispiel 19

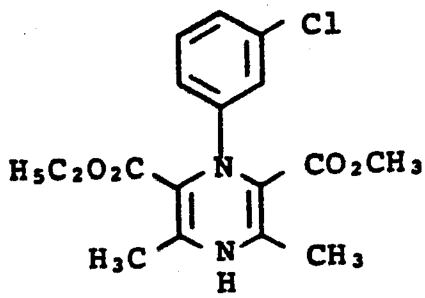

Analog Beispiel 1 wurde durch Umsetzung von 2-(3-Chloranilino)-acetessigsäureethylester-methoxycarbonylhydrazon mit 3-(Methoxycarbonylazo)-crotonsäuremethylester in Ethanol der 1.4-Dihydro-2.6-dimethyl-4-(3-chlorphenyl)-pyrazin-3.5-dicarbonsäure-ethyl-methyl-ester vom Fp: 196°C erhalten.
Ausbeute: 19 %.

Beispiel 20

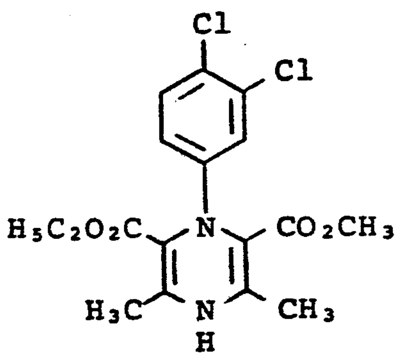

Analog Beispiel 1 wurde durch Umsetzung von 2-(3.4-Dichloranilino)-acetessigsäuremethylester-methoxycarbonylhydrazon mit 3-(Methoxycarbonylazo)-crotonsäureethylester in Ethanol der 1.4-Dihydro-2.6-dimethyl-4-(3.4-dichlorphenyl)-pyrazin-3.5-dicarbonsäure-ethyl-methyl-ester vom Fp: 220°C (Ethanol) erhalten.
Ausbeute: 18 %.

Beispiel 21

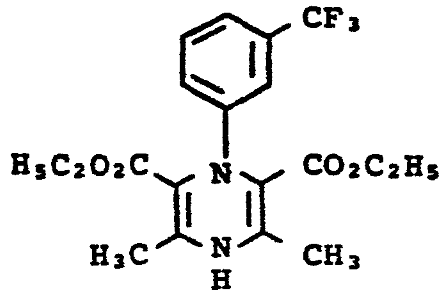

Analog Beispiel 2 wurde durch Umsetzung von 3-Trifluormethylanilin mit 3-(Methoxycarbonylazo)-crotonsäureethylester in Ethanol der 1.4-Dihydro-2.6-dimethyl-4-(3-trifluormethylphenyl)-pyrazin-3.5-dicarbonsäurediethylester vom Fp: 216°C erhalten.
Ausbeute: 20 %.

Beispiel 22

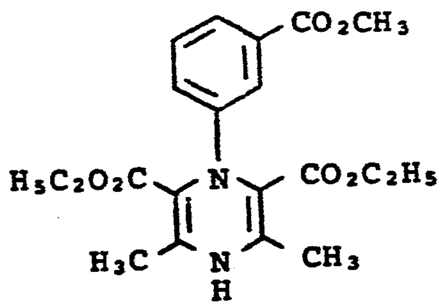

Analog Beispiel 1 wurde durch Umsetzung von 2-(3-Methoxycarbonylanilino)-acetessigsäureethylester-methoxycarbonylhydrazon mit 3-(Methoxycarbonylazo)-crotonsäureethylester in Ethanol der 1.4-Dihydro-2.6-dimethyl-4-(3-methoxycarbonylphenyl)-pyrazin-3.5-dicarbonsäurediethylester vom Fp: 186°C (Ethanol) erhalten.
Ausbeute: 15 %.

Beispiel 23

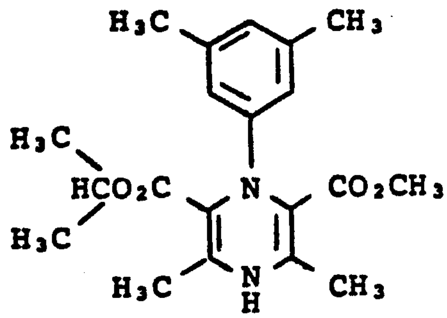

Analog Beispiel 1 wurde durch Umsetzung von 2-(3.5-Dimethylanilino)-acetessigsäuremethylester-methoxycarbonylhydrazon mit 3-(Methoxycarbonylazo)-crotonsäureisopropylester in Ethanol der 1.4-Dihydro-2.6-dimethyl-4-(3.5-dimethylphenyl)-pyrazin-3.5-dicarbonsäure-isopropylmethyl-ester vom Fp: 169°C erhalten.
Ausbeute: 21 %.

Beispiel 24

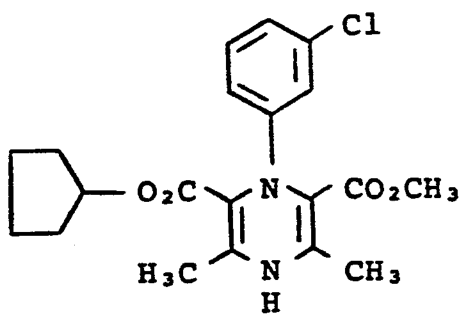

Analog Beispiel 1 wurde durch Umsetzung von 2-(Chloranilino)-acetessigsäuremethylester-methoxycarbonylhydrazon mit 3-(Methoxycarbonylazo)-crotonsäurecyclopentylester in Ethanol der 1.4-Dihydro-2.6-dimethyl-4-(3-chlorphenyl)-pyrazin-3.5-dicarbonsäure-cyclopentylmethyl-ester vom Fp: 151°C erhalten.
Ausbeute: 18 %.

 0149237

Patentansprüche:

1. 1.4-Dihydropyrazine der allgemeinen Formel I

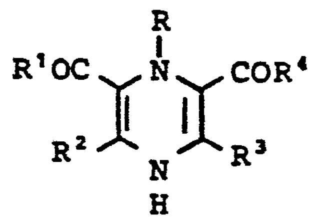

I

in welcher

R für carbocyclisches Aryl oder für Heterocyclen aus der Gruppe Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Benzthiadiazolyl, Chinolyl, Isochinolyl, Chinazolyl oder Chinoxalyl steht, wobei der Arylrest sowie die Heterocyclen gegebenenfalls 1 bis 3 gleiche oder verschiedene Substituenten aus der Gruppe Phenyl, Alkyl, Alkoxy, Alkylen, Dioxyalkylen, Halogen, Polyfluoralkyl, Polyfluoralkoxy, Alkylamino, Nitro, Cyano, Azido, Alkoxycarbonyl, Carbamoyl, Sulfamoyl oder $SO_m$-Alkyl (m = 0 bis 2) enthalten,

$R^1$ und $R^4$ gleich oder verschieden sind und

a) jeweils für eine Amino-, Mono- oder Dialkylaminogruppe stehen, wobei die Alkylgruppen

gegebenenfalls durch einen Phenylrest substituiert sein können, oder

b) jeweils für einen gegebenenfalls durch Alkyl, Alkoxy oder Halogen substituierten Anilinorest stehen, oder

c) für die Reste $OR^5$ oder $OR^6$ stehen, wobei $R^5$ und $R^6$ für einen geradkettigen, verzweigten oder cyclischen, gesättigten oder ungesättigten Kohlenwasserstoffrest stehen, der gegebenenfalls durch ein Sauerstoff- oder Schwefelatom in der Kette unterbrochen ist, und/oder der gegebenenfalls substituiert ist durch Halogen, Cyano, Hydroxy, Nitro, Nitrooxy, Alkoxycarbonyl, Phenyl, Phenoxy, Phenylthio, Phenylsulfonyl, Pyridyl oder eine Aminogruppe, wobei diese Aminogruppe durch zwei gleiche oder verschiedene Substituenten aus der Gruppe Alkyl, Alkoxyalkyl, Aryl oder Aralkyl substituiert ist, oder wobei die Aminogruppe in der Weise substituiert ist, daß 2 Substituenten mit dem Stickstoffatom einen 5 bis 7 gliedrigen Ring bilden, der als weiteres Heteroatom Sauerstoff oder Schwefel oder eine N-Alkyl/Phenyl-Gruppierung enthalten kann,

und

$R^2$ und $R^3$ gleich oder verschieden sind und jeweils für Wasserstoff, einen geradkettigen oder verzweigten, gegebenenfalls substituierten Alkylrest, einen Arylrest oder einen Aralkylrest stehen.

2. 1.4-Dihydropyrazine der allgemeinen Formel I, in welcher

R für einen Phenyl- oder Naphthylrest oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisooxazolyl, Benzoxadiazolyl, Benzthiadiazolyl, Chinolyl, Isochinolyl, Chinazolyl oder Chinoxalyl steht, wobei die genannten Heterocyclen sowie der Phenylrest und der Naphthylrest 1 bis 3 gleiche oder verschiedene Substituenten tragen können, wobei als Substituenten Phenyl, geradkettiges oder verzweigtes Alkyl mit bis zu 8 C-Atomen, Cycloalkyl mit 3 bis 7 C-Atomen, Alkenyl oder Alkinyl mit 2 bis 6 C-Atomen, Alkoxy mit 1 bis 4 C-Atomen, Alkenoxy und Alkinoxy mit 2 bis 6 C-Atomen, Tri-, Tetra- und Pentamethylen, Dioxymethylen, Dioxyethyliden, Halogen wie Fluor, Chlor, Brom oder Jod, Trifluormethyl, Trifluorethyl, Trifluormethoxy, Difluormethoxy, Tetrafluorethoxy, Dialkylamino mit 1 bis 4 C-Atomen, Nitro, Cyano, Azido, Alkoxycarbonyl

mit 1 bis 4 C-Atomen im Alkoxyrest, Carbamoyl, N.N-Dimethylcarbamoyl, Sulfamoyl oder $SO_m$-Alkyl, worin m eine Zahl von 0 bis 2 bedeutet und Alkyl 1 bis 4 C-Atome enthält, aufgeführt seien,

$R^1$ und $R^4$ gleich oder verschieden sind und jeweils für Amino-, Mono- oder Dialkylaminogruppen mit bis zu 4 C-Atomen je Alkylgruppe oder Dibenzylaminogruppe stehen

oder

für einen Anilinorest der durch Fluor, Chlor oder Alkyl mit 1 bis 4 Kohlenstoffatomen substituiert ist stehen,

oder

für die Reste $OR^5$ oder $OR^6$ stehen, wobei $R^5$ und $R^6$ gleich oder verschieden sind und für einen geradkettigen, verzweigten oder cyclischen, gesättigten Kohlenwasserstoffrest mit bis zu 10 C-Atomen stehen, der gegebenenfalls durch ein Sauerstoff in der Kette unterbrochen ist und/oder der gegebenenfalls substituiert ist durch Fluor, Chlor, Cyano, Hydroxy, Nitro, Alkoxycarbonyl mit 1 bis 4 C-Atomen in der Alkoxygruppe oder Amino, wobei diese Aminogruppe zwei gleiche oder verschiedene Substituenten aus der Gruppe Alkyl mit 1 bis 4

C-Atomen, Phenyl, Benzyl oder Phenethyl trägt oder wobei der Stickstoff dieser Aminogruppe mit den Substituenten einen 6-gliedrigen Ring bildet, der als weiteres Heteroatom eine N-Alkylgruppe mit 1 bis 4 C-Atomen im Alkylrest enthalten kann, und

$R^2$ und $R^3$ gleich oder verschieden sind und für Wasserstoff, Methyl, Ethyl, Phenyl oder Benzyl stehen.

3. 1.4-Dihydropyrazine der allgemeinen Formel I, in welcher

R für Phenyl, Benzoxadiazolyl, Benzthiadiazolyl oder Pyridyl steht, wobei der Phenylrest gegebenenfalls 1 bis 3 gleiche oder verschiedene Substituenten aus der Gruppe Methyl, Methoxy, Fluor, Chlor, Trifluormethyl, Trifluormethoxy, Nitro, Cyano, Azido oder Methoxycarbonyl enthält,

$R^1$ und $R^4$ gleich oder verschieden sind und jeweils für den Rest $OR^5$ oder $OR^6$ stehen, wobei $R^5$ und $R^6$ gleich oder verschieden sind und für einen geradkettigen, verzweigten oder cyclischen Kohlenwasserstoffrest mit bis zu 10 C-Atomen stehen, der gegebenenfalls durch ein Sauerstoff in der Kette unterbrochen ist oder der gegebenenfalls substituiert ist durch Fluor,

Chlor, Cyano, Hydroxy oder Nitro und

$R^2$ und $R^3$ gleich oder verschieden sind und jeweils für Methyl oder Benzyl stehen.

4. Verfahren zur Herstellung von 1.4-Dihydropyrazine der allgemeinen Formel I,

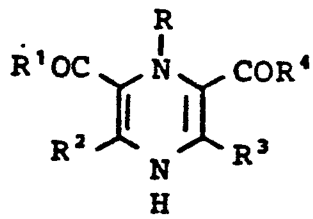

I

in welcher

R für carbocyclisches Aryl oder für Heterocyclen aus der Gruppe Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Benzthiadiazolyl, Chinolyl, Isochinolyl, Chinazolyl oder Chinoxalyl steht, wobei der Arylrest sowie die Heterocyclen gegebenenfalls 1 bis 3 gleiche oder verschiedene Substituenten aus der Gruppe Phenyl, Alkyl, Alkoxy, Alkylen, Dioxyalkylen, Halogen, Polyfluoralkyl, Polyfluoralkoxy, Alkylamino, Nitro, Cyano, Azido, Alkoxycarbonyl, Carbamoyl, Sulfamoyl oder $SO_m$-Alkyl (m = 0 bis 2) enthalten,

0149237

$R^1$ und $R^4$ gleich oder verschieden sind und

a) jeweils für eine Amino-, Mono- oder Dialkylaminogruppe stehen, wobei die Alkylgruppen gegebenenfalls durch einen Phenylrest substituiert sein können, oder

b) jeweils für einen gegebenenfalls durch Alkyl, Alkoxy oder Halogen substituierten Anilinorest stehen, oder

c) für die Reste $OR^5$ oder $OR^6$ stehen, wobei $R^5$ und $R^6$ für einen geradkettigen, verzweigten oder cyclischen, gesättigten oder ungesättigten Kohlenwasserstoffrest stehen, der gegebenenfalls durch ein Sauerstoff- oder Schwefelatom in der Kette unterbrochen ist, und/oder der gegebenenfalls substituiert ist durch Halogen, Cyano, Hydroxy, Nitro, Nitrooxy, Alkoxycarbonyl, Phenyl, Phenoxy, Phenylthio, Phenylsulfonyl, Pyridyl oder eine Aminogruppe, wobei diese Aminogruppe durch zwei gleiche oder verschiedene Substituenten aus der Gruppe Alkyl, Alkoxyalkyl, Aryl oder Aralkyl substituiert ist, oder wobei die Aminogruppe in der Weise substituiert ist, daß 2 Substituenten mit dem Stickstoffatom einen 5 bis 7 gliedrigen Ring bilden, der als weiteres Heteroatom Sauerstoff oder Schwefel oder eine N-Alkyl/Phenyl-Gruppierung enthalten kann,

und

$R^2$ und $R^3$ gleich oder verschieden sind und jeweils für Wasserstoff, einen geradkettigen oder verzweigten, gegebenenfalls substituierten Alkylrest, einen Arylrest oder einen Aralkylrest stehen,

dadurch gekennzeichnet, daß man Hydrazone von α-Amino-ß-ketocarbonsäurederivaten der allgemeinen Formeln II und IV

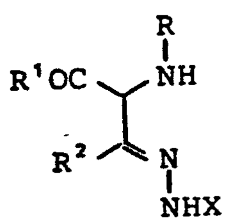

II

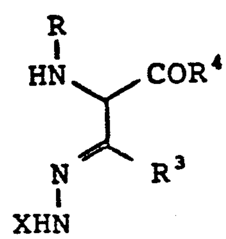

IV

in welchen R, $R^1$, $R^2$, $R^3$ und $R^4$ oben angegebene Bedeutung haben und

X für eine Carbamoyl- oder eine Alkoxycarbonylgruppe steht,

mit Azo-en-carbonylverbindungen der allgemeinen Formeln III und V

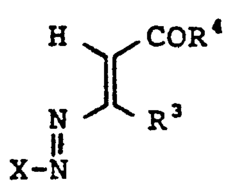

III

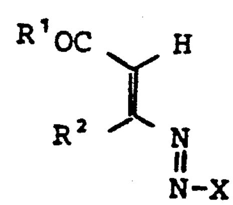

V

in welchen R, $R^1$, $R^2$, $R^3$ und $R^4$ die oben angegebene Bedeutung haben und

X für eine Carbamoyl- oder eine Alkoxycarbonylgruppe steht,

gegebenenfalls in Gegenwart inerter organischer Lösungsmittel bei Temperaturen zwischen 20 und 150°C umsetzt.

5. Verfahren zur Herstellung von 1.4-Dihydropyrazinen der allgemeinen Formel VI

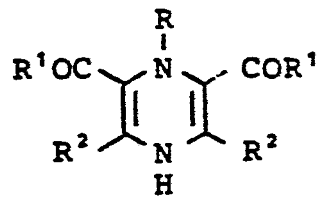

VI

die mit den Verbindungen der allgemeinen Formel I identisch sind für die Bedingung, daß der Substituent $R^1$ mit dem Substituenten $R^4$ identisch ist und der Substituent $R^2$ mit dem Substituenten $R^3$ identisch ist und in welcher

die Substituenten R, $R^1$, $R^2$, $R^3$ und $R^4$ die im Anspruch 4 angegebene Bedeutung haben,

dadurch gekennzeichnet, daß man ein primäres Amin der allgemeinen Formel VII

$$R\text{-}NH_2 \qquad VII$$

in welcher R die oben angegebene Bedeutung hat,

mit Azo-en-carbonylverbindungen der allgemeinen Formel V (bzw. III),

in welcher $R^1$ und $R^2$ (bzw. $R^3$ und $R^4$) und X die oben angegebene Bedeutung haben,

gegebenenfalls in Gegenwart inerter organischer Lösungsmittel bei Temperaturen zwischen 20 und 150°C umsetzt.

6. 1.4-Dihydropyrazine der allgemeinen Formel I gemäß Anspruch 1 zur Verwendung bei der Bekämpfung von Erkrankungen.

7. 1.4-Dihydropyrazine der allgemeinen Formel I gemäß Anspruch 1 zur Verwendung bei der Bekämpfung von Kreislauferkrankungen.

8. Arzneimittel enthaltend mindestens ein 1.4-Dihydropyrazin der allgemeinen Formel I gemäß Anspruch 1.

9. Verwendung von 1.4-Dihydropyrazinen der allgemeinen Formel I gemäß Anspruch 1 bei der Bekämpfung von Kreislauferkrankungen.

10. Verfahren zur Herstellung von Arzneimitteln, dadurch gekennzeichnet, daß man 1.4-Dihydropyrazine der allgemeinen Formel I gemäß Anspruch 1, gegebenenfalls unter Verwendung von Hilfs- und Trägerstoffen in eine applizierbare Form überführt.